# EUROPEAN PATENT SPECIFICATION

(11) **EP 0 294 595 B1**
(45) Date of publication and mention of the grant of the patent: **24.01.1996**
(21) Application number: 88107311.8
(22) Date of filing: 06.05.1988
(51) Int. Cl.: C12N 15/70

(54) **Polynucleotide composition and method for its production**
Polynucleotid-Zusammensetzung und Verfahren zu ihrer Herstellung
Composition polynucléotidique et méthode pour sa production

(30) Priority: 08.05.1987 US 47816
(43) Date of publication of application: 14.12.1988
(73) Proprietor: ENZO BIOCHEM, INC., New York, N.Y. 10013 (US)
(72) Inventor: Yang, Huey-Lang, Tenafly New Jersey 07670 (US); Donegan, James J., New York, N.Y. 10021 (US)
(74) Representative: VOSSIUS & PARTNER

(56) References cited:
- EP-A- 0 237 737
- WO-A-87/02702
- JOURNAL OF CLINICAL MICROBIOLOGY, vol. 24, no. 1, July 1986, pages 16-20, American Society for Microbiology, Washington, DC, US; R.F. AMBINDER et al.: "The vector homology problem in diagnostic nucleic acid hybridization of clinical specimens"

## Description

This invention relates to specific polynucleotide compositions, to methods for producing them and to their use.

Polynucleotide preparations generally comprise a nucleotide sequence that is specifically of interest and other polynucleotides which are not of interest and can, in fact, interfere with the use of the polynucleotide of interest These preparations are conventionally obtained as follows. A copy of the desired polynucleotide is inserted into a vector, such as a bacterial plasmid, resulting in a recombinant vector. The recombinant vector is then introduced into a host, such as a bacterium, resulting in a transformed host where it replicates. After a period of growth permitting plasmid replication of the recombinant vector, the multiple copies are isolated, and, if necessary, the desired polynucleotides are excised. These polynucleotide preparations have use, inter alia, in therapeutic and diagnostic applications.

In this conventional method the preparation containing the desired polynucleotides generally also contains other polynucleotides which are not of interest These polynucleotides arise from two sources., the conventional vectors used for cloning, such as pBR322, and the host chromosomes. When an isolate containing the desired polynucleotides is labelled, these other polynucleotides are labelled as well. Many of these other polynucleotides are complementary to polynucleotides of commensal bacteria that are often found in biological samples. Thus, these other labelled polynucleotides can react with the commensal bacteria in the sample producing false positives.in diagnostic applications. These drawbacks are discussed in R.F. Ambinder et.al., "The Vector Homology Problem in Diagnostic Nucleic Acid Hybridization of Clinical Specimens," J.Clin.Microb., 24 16-20 (1986).

An example is where the insert is specific for human papilloma virus (HPV) and is cloned into a pUC9 vector. The vector is introduced into an E. coli cell and the E. coli is allowed to replicate. Vector polynucleotides containing the HPV insert are isolated from the E. coli bacteria by any one of several methods known in the art. The HPV insert is cleaved with restriction enzymes, purified, and labelled, for example, with a fluorescent molecule by means of nick translation. When this HPV probe is tested against two different vaginal smears, one that contains the HPV and one that does not, both tests can appear positive because E. coli bacteria often inhabit the female urinary tract. The false positive obtained with the HPV negative vaginal smear is a result of non-specific polynucleotides present in or with the HPV probes that were labelled during the labelling of the HPV-specific polynuleotides and are complementary to polynucleotide sequences found either in the plasmids or chromosomes of E. coli. The abundance of E. coli cells in the urinary tract of the HPV-negative sample more than compensates for the low levels of labelled E. coli polynucleotides present with the probe.

One way to produce desired polynucleotides that are not complementary to E. coli polynucleotides is to produce the polynucleotides synthetically, for example, by means of a gene-synthesizer instrument. Currently available instruments are capable of producing polynucleotides of about 50 nucleotides (0.50 kb). However, a polynucleotide prepared in this way cannot be labelled to the extent that a longer (1-2 kb) sequence can. A long probe will generally give a greater signal upon hybridizing to a target polynucleotide than will a short probe.

Furthermore, a hybrid formed between long stretches of polynucleotides is significantly more thermodynamically stable than a hybrid formed between short stretches of polynucleotides. This allows the hybrid to remain intact at high temperatures and under high stringency conditions, and simplifies the denaturation of randomly formed hybrids.

Thus, a need exists, particularly in hybridization assays, for a method of creating homogeneous nucleotide preparations, particularly those which provide sequences longer than those currently available from genesynthesizer instrumentation.

To address this need the present invention provides a composition comprising a specific oligo- or polynucleotide of interest that is free of non-specific oligo- or polynucleotides other than the one of interest. This oligo- or polynucleotide can be a sequence many times longer than could be achieved by gene synthesizer instrumentation and is of a homogenity greater than previously available from other in vitro or in vivo techniques. Such homogeneous oligo- or polynucleotides are advantageous for use, inter alia in preparing hybridization assay reagents.

Accordingly, the invention provides a composition comprising a specific oligo- or polynucleotide of interest free of non-specific oligo- or polynucleotides. This composition is prepared by a method which comprises the steps of (i) forming a vector containing the oligo- or polynucleotide insert of interest, (ii) replicating said insert in a suitable cell or cell free system, (iii) rendering any oligo- or polynucleotide not associated with the insert incapable of annealing, and (iv) recovering said insert as the specific oligo- or polynucleotide composition. The cell or cell free system is a host bacterial cell and the oligo- or polynucleotide is rendered incapable of annealing by selective inactivation or digestion. The vector is a lethal phage and the oligo-or polynucleotide is inactivated thereby. This lethal phage is a lytic phage, i.e. the phage vectors T7-T102, T7-T104 and T7-T105.

The invention further provides a composition prepared by the method described above. The composition comprises a specific oligo- or polynucleotide of interest and free of non-specific oligo- or polynucleotide. The composition is particularly suitable for labelling with any of the labelling systems known for hybridization or other specific binding systems, and provides reagents of exceptional specificity for uses including hybridization assay, drug delivery and gene regulation.

The recombinant vector further incorporates at least one component of a labelling system. This labelling system component can be incorporated with at least one nucleotide of the oligo- or nucleotide sequence or of the phage or of both. Exemplary labelling system components include biotin and its analogs, enzymes, fluors, radiolabels and the like.

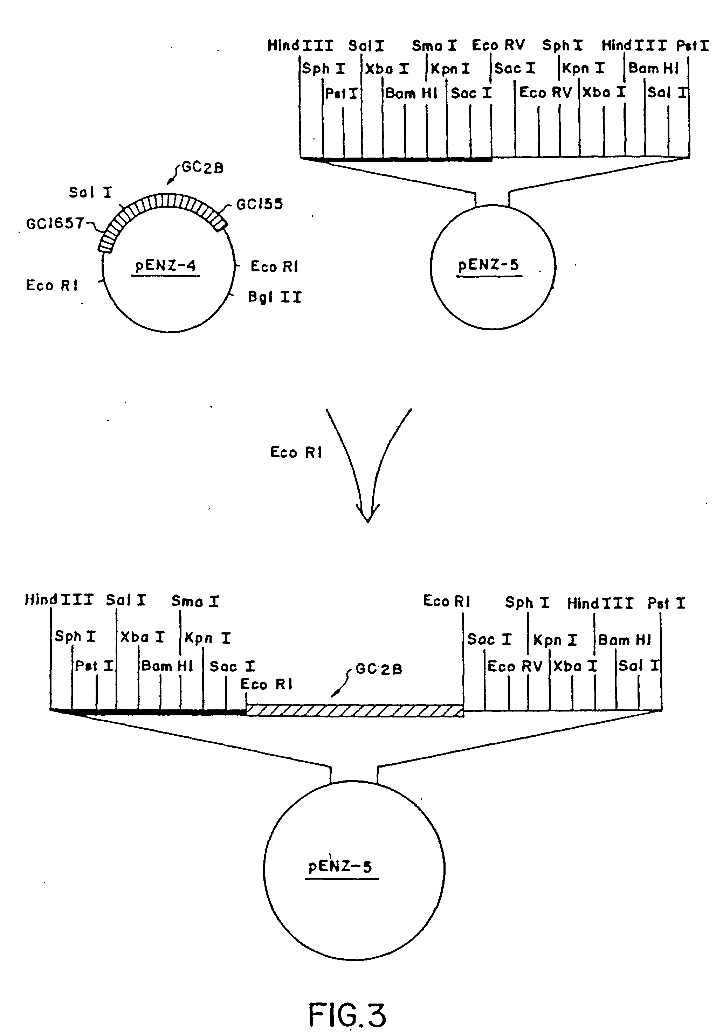

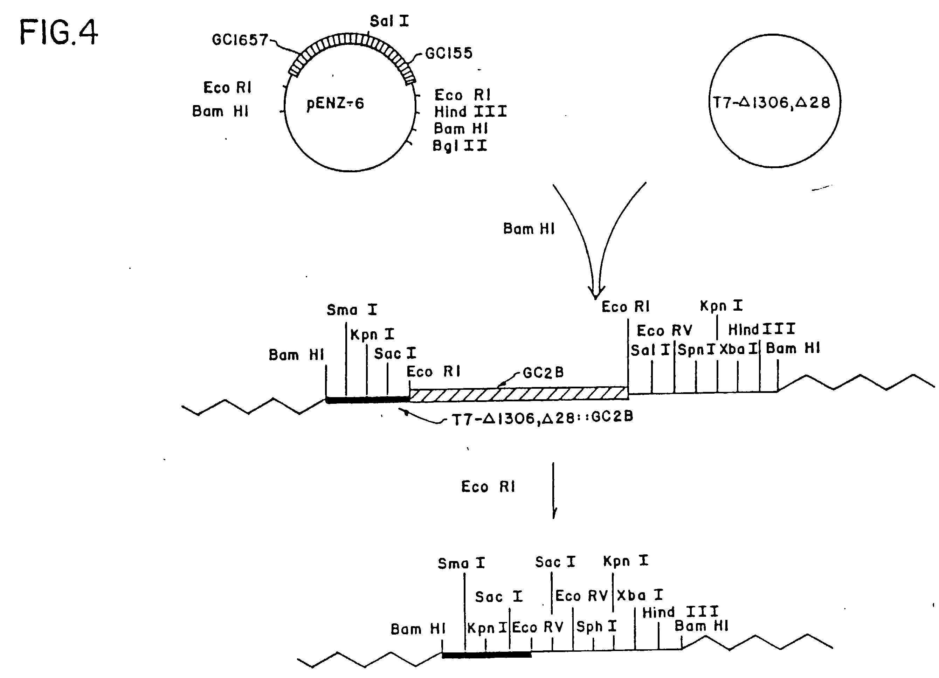

Thus, the present invention is directed to a reagent composition for determining the presence or absence of a target nucleic acid in a sample, said composition comprising:
(a) a nucleic acid construct consisting essentially of:
   (i) a nucleic acid vector which is a bacteriophage selected from the group consisting of T7-T102 which is a derivative of T7 having deletions spanning the regions from 836 to 3142 and from 11293 to 11570 base pairs of the T7 genome and having the restriction sites shown in Fig. 4; T7-T104 which is a derivative of T7 having deletions spanning the regions from 836 to 3142, from 5845 to 7879 and from 11293 to 11570 base pairs of the T7 genome and having the insert GC2B consisting of the DNA sequence inserts GC155 and GC1657 from N. gonorrhoeae as shown in Fig. 3; and T7-T105 which corresponds to T7-T104 wherein the insert GC2B has been deleted;
   (ii) a segment of nucleic acid, which segment is sufficiently homologous to said target to hybridize substantially only with that target sequence and not with other sequences that may be present as contaminants in said sample; and
   (iii) at least one labeling system component which is required for production of a detectable signal and is covalently bound to at least one of (i) or (ii);

   and
(b) at least one additional component of said labeling system which is bindable with said labeling system component (iii) and renders said labeling system detectable (iii) when said labeling system component is not itself detectable.

Fig. 1 illustrates the overall series of reactions described in Example 2 for the preparation of plasmid vectors pENZ-3, pENZ-4, pENZ-5 and pENZ-6.

Fig. 2 illustrates in more detail the fusion of the polylinker of vectors mp19 and tg130, resulting in a plasmid vector identified as pENZ-5.

Fig. 3 illustrates in more detail the transfer of the GC2B insert from pENZ-1 into pENZ-5, resulting in plasmid vector pENZ-6.

Fig. 4 illustrates the construction of T7-Δ1306,Δ28::GC2B from pENZ-6 and T7- Δ1306, Δ28 and the deletion of the GC2B segment therefrom to form vector T7-T102.

As previously noted, this invention discloses a composition and method for producing specific polynucleotide preparations, that is preparations which are free of polynucleotide sequences that hybridize to non-specific polynucleotides.

The polynucleotide of interest usually comprises at least fifty nucleotides whose sequence is unique to a target sequence, usually unique to or characteristic of a specific organism or specific group of organisms. Polynucleotide segments obtained from such an insert are considered specific polynucleotides if they comprise nucleotides of a sequence sufficient to uniquely hybridize with a target sequence of the organism. The target can be narrow or broad and encompass from a single species of a genus to a number of species from several genera. Such specific polynucleotides can thus be used as probes to detect the presence of a target organism or tissue, or as a therapeutic entity or as part of a drug delivery system.

### Selection and Use of Lytic Phage

The invention uses a lethal phage, that is a lytic phage which destroys the viability of a host bacterium it has infected. A vector derived from a lethal phage will not have any homology to any polynucleotides of the host chromosome. The phage generally replicates to produce about 50-500 phage particles per host cell and concurrently degrades the host polynucleoties. Accordingly, the only remaining intact oligo- or polynucleotide are those of the vector and the insert of interest.

In accordance with the invention, the polynucleotides of phage selected to be used as vectors are isolated, cut by means of a predetermined restriction endonuclease to provide an insertion site and, then, specific polynucleotides of interest are introduced to produce recombinant polynucleotides.

The recombinant polynucleotides are packaged and, with standard methods using suitable host cells, stocks of the phage are produced. An appropriate host cell is selected in which the phage is allowed to replicate. E. coli is a preferred host cell.

The recombinant polynucleotides of the desired phage are removed from their capsid by phenol treatment. The recombinant polynucleotides can then be directly labelled and used. unlike with conventional vectors. Alternatively, the insert can be re::overed along with a portion of the vector sequence which can then serve as one component of a signal generating system. Or, if desired, it can be enzymatically treated to cleave the specific polynucleotide inserts. In this case, the inserts are isolated using standard methods and the specific polynucleotides alone are labelled. Methods to achieve this labelling are known to those skilled in the art.

A probe made from a recombinant polynucleotide in accordance with the invention will not contain any polynucleotides complementary to the bacterial host chromosomal polynucleotides. Because the lethal phage have a mechanism to destroy the viability of their bacterial hosts after a period of propagation, the host chromosomes will no longer replicate. In addition, the phage generally degrade the host polynucleotides so that if any integration between the phage polynucleotides and the host chromosome would have naturally occurred, the resulting integration product is destroyed. Nevertheless, to ensure absolute purity, the phage can be separated from any residual host polynucleotides in the mixture by means of a cesium chloride gradient. and/or by subjecting the mixture to nuclease treatment using deoxyribonuclease, ribonuclease or both The nuclease only degrades the naked polynucleotides but not the phage.

Since the probes will not contain any polynucleotides complementary to polynucleotides found either in the bacterial host chromosomes or in the plasmids carried in such hosts, the probe will not hybridize to non-specific polynucleotides in biological samples.

The invention makes use of certain preferred lytic phages, i.e. derivatives of T phages. The T7 double strand DNA bacteriophage, has been reviewed at length. See Dunn and Studier, Complete Nucleotide Sequence of Bacteriophage T7 DNA and the Loctations of T7 Genetic Elements, J. Mol. Biol., 166; 477-535 (1983).

Because the sequence and genetic function of the entire genome of T7 is known, it has advantages for use as a vector. The advantages are due to the fact that there are a number of nonessential genes present, the removal of which does not affect the viability of the phage in appropriate hosts. For example, there are genes that code for proteins that eliminate restriction enzyme activities of the host that would otherwise destroy the T7 phage. If host bacteria which do not contain such restriction enzymes are used (restriction minus), the genomic regions in the T7 phage coding for those proteins are not needed. The phage DNA must be of a certain size to be packaged into an infective particle. Generally, the size of the DNA in the resulting particle cannot be expanded beyond about 10% or be decreased beyond about 20%. This then places a limit on the size of the inserts. However, if non-essential genes are deleted from the phage, then larger inserts can be integrated.

The products can include, for example, the original T7 DNA, and the specific nucleotide sequence as an insert, (the recombinant polynucleotide). This DNA molecule needs to be packaged with a protein coat so that it can infect E. coli cells. A suitable method for preparing packaging proteins is described by Kuemmerle and Masker, Virol. 23: 509 (1977). The method involves the use of mutant strains of T7 phages in appropriate suppression-negative E. coli hosts to provide the packaging protein.

The ligation mixture of T7 DNA and insert DNA are incubated with the extract along with all additional necessary components which are known to one skilled in the art. After sufficient time has elapsed for the packaging to occur, the mixture is added to a culture of host cells and melted agar and the slurry is poured onto a broth agar plate. The plate is incubated at 37° C until plaques are formed. The plaques containing the specific polynucleotides, namely, the probe phages, are then determined. One method for detecting the presence of the specific polynucleotides in any plaque is by making plaque lifts and incubating the T7 polynucleotides from each plaque with a probe comprising the specific polynucleotides. See, for example, Maniatis, et al., Molecular Cloning, Cold Spring Harbor Laboratory (1982). The presence of the specific polynucleotides can also be determined by: (i) growing up phage stocks from a number of plaques; (ii) isolating the polynucleotides from the plaques by phenol extraction/ethanol precipitation; and (iii) using appropriate restriction enzymes to identify the presence of the specific polynucleotides in the phage.

After the desired phage plaques are identified, purified phage stocks are obtained therefrom by serial dilution. The phage are recovered and cultured, plaques are isolated and phage stock prepared therefrom. These phage stocks are then used to prepare large-scale quantities of phage by adding an aliquot thereof to a liquid culture of host cells and incubating the culture until lysis occurs. The phages are purified from possible residual host chromosomal polynucleotides by cesium chloride centrifugation and/or nuclease treatment.

The polynucleotides of the host cell must be independently inactivated to separate all the host cell polynucleotides from the polynucleotides of interest during purification. Therefore in conventional techniques these host polynucleotides contaminate preparations of the polynucleotide of interest.

One method for inactivating the host cell polynucleotides is by nuclease treatment, as shown above. Another method is to irreversibly cross-link them. This method requires that the polynucleotides of the host cell are essentially in double-stranded form. A number of reagents are available for cross-linking double-stranded polynucleotides. However, the reagent must be one that cannot penetrate the phage protein coat such that the polynucleotides of the phage would also be inactivated.

A variation of this method is to attach an intercalating agent, which has the ability to cross link double-stranded polynucleotides to a bead. The bead can be any polymer, for example, Sepharose®, agarose, or silica. The intercalating agent is attached to the bead by derivatizing the intercalating agent so that it can react with the bead and yet leave the functionalities on the intercalating agent required for cross-linking intact. An example of such a reagent is psoralen, an intercalating and a cross-linking agent. Psoralen can be derivatized to contain, for example, an isothiocyanate group which will react with agarose.

Following the attachment of the psoralen to the beads, the beads are mixed with a cell extract comprising the packaged phage particles and the naked polynucleotides of the host cell. The mixture is stirred for a predetermined time interval. The psoralen can intercalcate only into the naked double-stranded polynucleotides, and not into the enclosed polynucleotides of the phage.

The mixture is then exposed to light of a wavelength which will activate the psoralen. The activated psoralen cross-links the polynucleotides into which it has intercalated. The beads are spun down at a low speed with a centrifuge leaving the phage particles suspended in the supernatant. The phage particles are then spun down at a high speed and lysed to release their polynucleotides, including ones comprising the specific polynucleotide.

The following examples illustrate but are not a limitation of the present invention.

### REFERENCE EXAMPLE I

The experiments reported in this example illustrate that commonly used cloning vectors, such as pBR322 or M13, cross-react with E. coli DNA, whereas the lethal phage, such as T7, does not cross-react with E. coli DNA.. This illustrates the proper selection of vectors for preparation of specific DNA sequence compositions suitable for use as hybridization probes.

To measure the cross reactivity of T7, pBR322 and M13 DNA with that of E. coli, p32 labelled DNA probes are hybridized with target DNA from clinical isolates (ten) of E. coli. The sources of the labelled probes are wild type T7 bacteriophage and two commonly utilized cloning vectors, pBR322 and M13. The E. coli used in this experiment are isolated from various clinical specimens and are considered to be examples of typical clinical isolates. At least four of the strains contain plasmids that confer antibiotic resistance.

Twelve DNA's, (the ten E. coli clinical isolates described above together with a laboratory E. coli strain JM 103 and calf thymus DNA) are prepared according to methods described by Maniatis, et al. supra. The DNA is dotted onto a Pall nylon membrane (Pall Biodyne) by adding TE buffer (10 mM Tris HCl, pH 8.0, 1mM EDTA) to each DNA sample (final concentration of 20 ug per 125 ul). One tenth volume (12.5 ul) of 2M NaOH is added to denature the DNA (room temperature for 10 min). Then, 7M ammonium acetate (150 ul, pH 8.0) is added followed by 1 M ammonium acetate (712.5 ul). DNA (i.e., 1 ug/50 ul), from each sample is dotted onto Pall nylon membrane using a dot-blot manifold with vacuum (10 inch Hg). Afterwards, membranes are rinsed briefly with 2X SSC (0.3 M NaC1, 0.03 M Na citrate, pH 8.0), air dried and baked under vacuum at 80°C for 2 hours before use.

The three DNA probes tested (T7, pBR322 and M13) are labelled with p32 by nick translation (see Maniatis, et al., supra p. 109) to provide probes having a specific activity of 5 x 10⁷cpm/ug of DNA.

The methods of prehybridization and hybridization used are as described in Maniatis, et al., supra. After hybridization at 65°C for 48 hours, membranes are washed 3 times with 2X SSC, 0.1% SDS and 3 times with 0.2X SSC, 0.1% SDS at 65°C (20 min for each wash). Filters are exposed to x-ray film (Kodak) and, after development, the areas of the dots are cut out and quantitated by scintillation counting. Results of this study are summarized in Table 1.

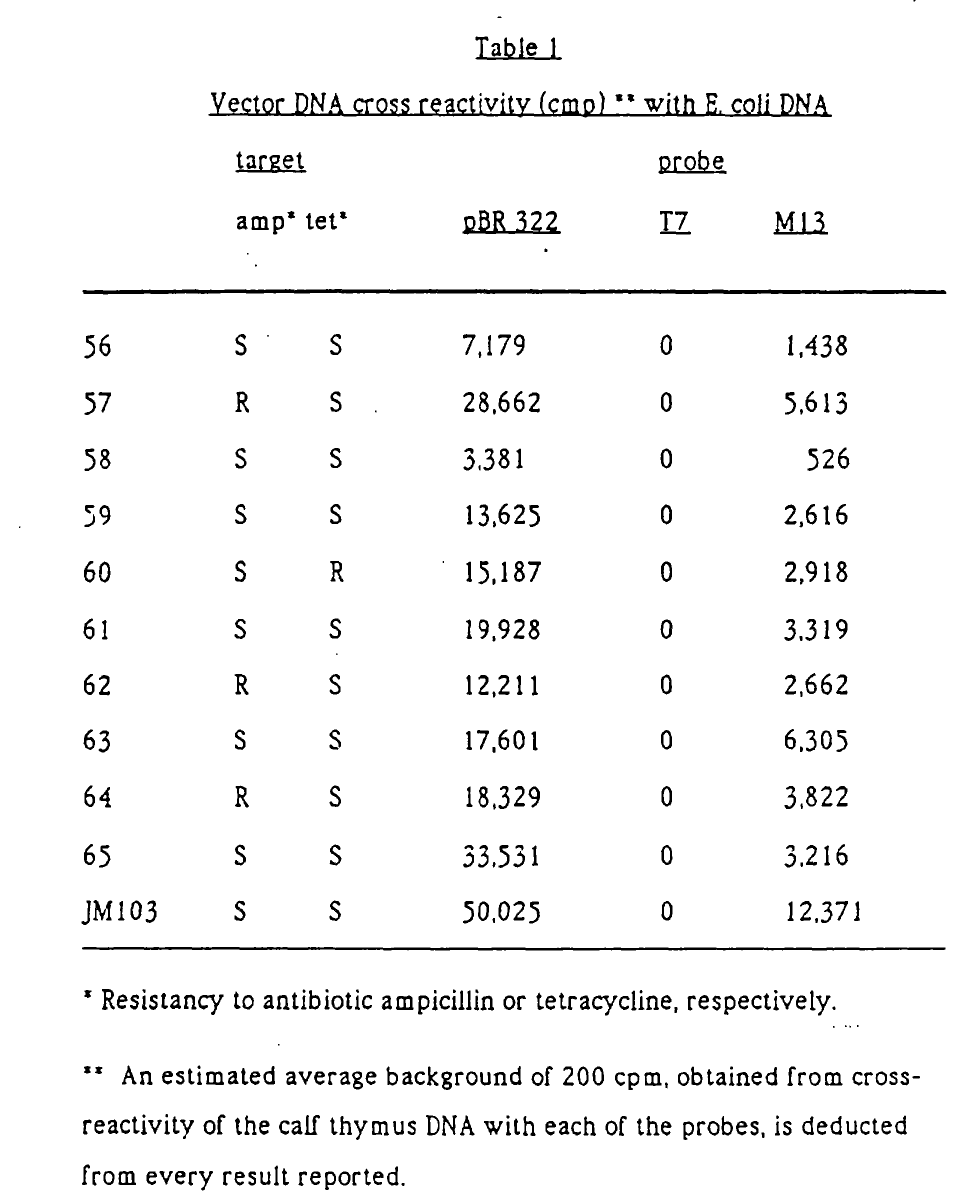

It can be seen from these data that the commonly used plasmid pBR322 is highly cross-reactive with E. coli, DNA, the M13 is also highly cross-reactive with E. coli DNA, but the lethal phage, exemplified by T7, are not cross-reactive with the E. coli tested. Thus, this suggests that lethal phage are highly desirable for use as cloning vectors to produce oligo- or polynucleotide preparations which are free of cross-reactive, non-specific sequences. This is highly desirable, inter alia, for use in hybridization probes.

### EXAMPLE 1

The experiments reported in this example relate to the construction of several recombinant lethal phage vectors. These vectors are then used by inserting and propogating species-specific DNA sequences in them. A wide variety of DNA sequences of interest are suitable for insertion into these vectors.

### Construction of pENZ-4

Background information is provided in Yang, et al., U.S. serial no. 823,921, filed on January 30, 1986 (EP-A-237737) and assigned to the instant assignee, which is hereby incorporated by reference. This describes recombinant bacterial strains deposited with the American Type Culture Collection as Accession Nos. 53411 and 53409. These contain bacteriophage vector mp8 (derived from bacteriophage M13) which contain DNA sequence inserts from Neisseria gonorrhoeae referred to as GC 155 and GC 1657, respectively. Hereafter they are referred to as pENZ-1 (mp8::GC155) and pENZ-2 (mp8::GC1657). For additional background on vector mp8, see Messing, J., Methods in Enzymology, 101; 20-78 (1983).

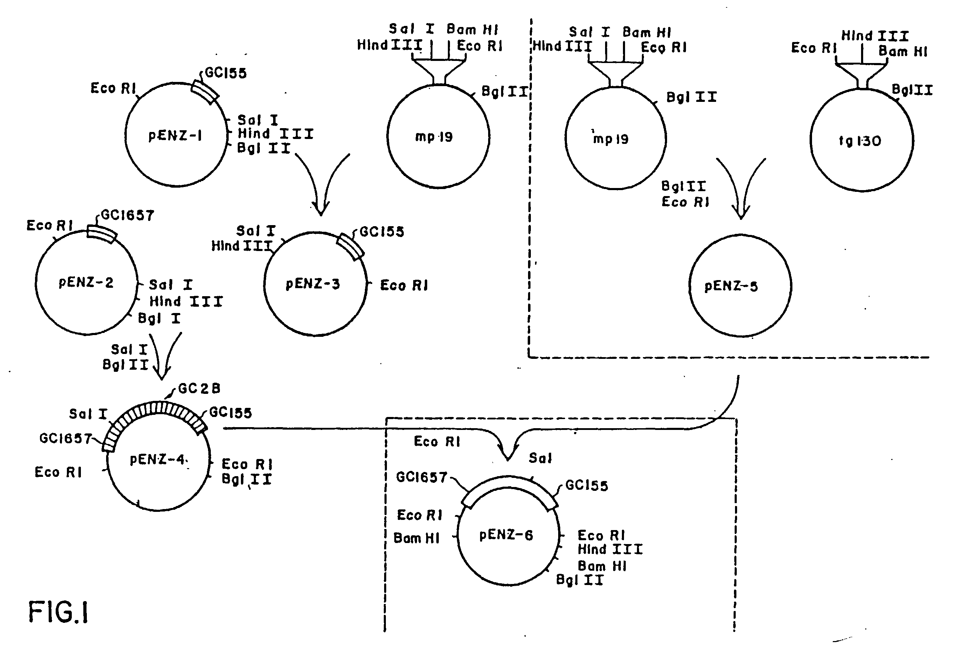

Referring now to Fig. 1, the GC 155 sequence which is removed from pENZ-1 by Eco R1 and Sal I digestion is introduced into bacteriophage vector mp19, also digested with Eco R1 and Sal I, to produce plasmid pENZ-3 (mp19::GC155). For additional background on mp19, see Yanisch-Perron, et al., Gene, 33: 103-119 (1958). The pENZ-3 plasmid is then used to transform E. coli strain JM 109 cells. The transformed JM 109 cells are cultured to provide a heterogeneous phage stock and supercoiled DNA is isolated therefrom. The pENZ-3 is then identified in the phage stock by restriction enzyme analysis. The pENZ-3 plasmid differs from the original pENZ-1 only in that the polylinker annealing results in the GC 155 segment being inserted in reverse orientation.

The pENZ-2 and pENZ-3 plasmids are digested with Sal I and Bgl II, and then ligated together to produce a plasmid containing both inserts (GC155 and GC1657). This is hereafter referred to as plasmid pENZ-4 and the segment containing both GC155 and GC1657 is referred to as GC2B (hatched portion).

### Construction of pENZ-5

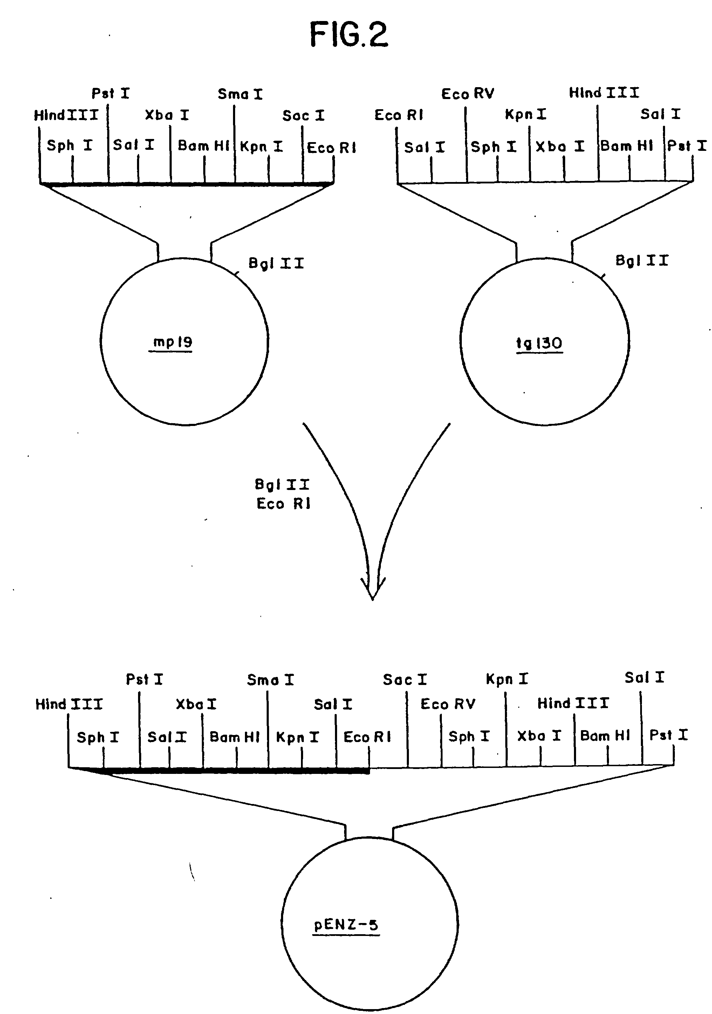

Continuing to refer to Fig. 1, vector mp19 and vector tg130, see Kieny, et al., Gene, 26; 91 (1983), are digested with Eco RI and Bgl II, and then ligated together to form plasmid pENZ-5. The plasmid so-produced is used to transform JM 109 cells and the pENZ-5 is identifed by restriction enzyme analysis from phage stock as described above. Plasmid pENZ-5 has an Eco R1 site flanked by Bam H1 sites. Also, in between these two Bam H1 sites, are the additional restriction sites Kpn I, Sma I, Sac I, Sph I, Xba I and Hind III. This is shown in more detail in Fig. 2.

### Construction of pENZ-6

It is desirable for pENZ-4, preparation of which is described above, to have Bam H1 sites at each end of the GC 155 plus GC 1657 (GC2B) insert.

Although this could be accomplished conventionally by the introduction of commercially available linkers at both ends of the insert, it is even more preferable to create a vector that has additional restriction sites available. It is most desirable to achieve these objectives while avoiding the additional manipulations conventionally required to add linkers compatible with other enzymes for each intended insertion. This combination of advantages is made possible by combining pENZ-4 and pENZ-5 described above, to form the plasmid pENZ-6.

Accordingly, and still referring to Fig. 1, pENZ-4 and pENZ-5 are digested with Eco R1, and then ligated together to form plasmid pENZ-6. The pENZ-6 so-produced is used to transform JM 109 cells and is identified by restriction enzyme analysis from phage stock as described above. The resulting pENZ-6, including N. gonorrhoeae insert GC2B and the relevent portion of its restriction map are shown in greater detail in Fig.3.

### Construction of T7-T101::GC2B

By way of background, bacteriophage T7-T101, used in this experiment, is a modification of bacteriophage T7, discussed in Dunn, et al., supra. It was first prepared by W. Studier, Brookhaven National Laboratory.

Essentially it is a T7 phage from which two segments have been deleted. Referring to the numbering in the complete T7 DNA sequence set forth in Dunn et. al., supra. the two segments deleted are base pairs 836 to 3142 (Δ 1306) and 11,293 to 11,570 (Δ28). Also, T7-T101 has a small linker sequence inserted into the edges of the Δ1306 deletion. This linker sequence comprises a Bgl II site flanked by two Bam H1 sites. Thus, T7-T101 is missing a total of 2,586 bp of the T7 DNA sequence and lacks genes that normally would be used for overcoming host restriction systems. As such, this phage is grown in E. coli strain BL 21, which is a restriction minus, modification minus (r-m-) derivative of E. coli B.

Referring now to Fig. 4, the pENZ-6 plasmid, prepared as described above, and the T7-T101 bacteriophage are digested with Eco R1 and then ligated together. The resulting product of ligation is packaged in accordance with the procedure described by Kuemmerle, et. al. In Vitro Packaging of UV Radiation-Damaged DNA from Bacteriophage T7, J. Virol. 23; 509-516 (1977). After completion of the packaging reaction, the resulting preparation is used to transform E. coli BL 21 cells, the cells are cultured and plaques are isolated. Phage stock is made from the individual plaques, DNA is isolated and restricticn enzyme analysis is carried out to identify T7-T101::GC2B, which is the packaged combination of the pENZ-6 and T101 sequences.

Still referring to Fig. 4, the GC2B insert from the pENZ-6 can be excised by digestion with any of Bam H1, Eco R1, Kpn I, Sac I or Xba I. Although the process described earlier makes it possible to put an insert with Eco R1 ends into a Bam H1 site, the T7-T101::GC2B has more available restriction sites than even the parental T7-T101 vector.

Therefore, the GC2B insert has been successfully used to introduce these additional restriction sites into T7-T101. The resulting T7-T101::GC2B is then used to produce a generally applicable vector which carries the polylinker introduced by GC2B, but without the GC155 or GC1657 sequences, as is described in more detail below.

### Construction of Vector T7-T102

Continuing to refer to Fig. 4, the GC2B insert is removed from the T7-T101::GC2B by Eco R1 digestion. The T7-T101::GC2B from which the GC2B insert has been excised is then treated with DNA ligase to religate together the left and right parts of the T7-T101::GC2B without the GC2B insert while still retaining the polylinker. The resulting product of ligation is packaged and used to transform E. coli BL 21 cells. These transformed cells are cultured, plaques isolated, and phage stock is made from the individual plaques. DNA is isolated and restriction enzyme analysis is carried out to identify the T7-T101::GC2B from which the GC2B insert has been excised. This is hereafter referred to as vector T7-T102, which is useful for insertion of a broad range of target specific DNA sequences. It should be noted that construction of vector T102 introduces novel restriction enzyme sites into the T7 genome, specifically sites for Sac I, Sma I, Eco R1, Bam H1, Eco RV, Pst I, Sph I and Hind III.

By way of review, not all of the coding capacity of the T7 genome is necessary for viability under appropriate circumstances. T7-T101 and T7-T102 are T7 vectors that both have deletions spanning the regions from 836 to 3,142 (Δ1306) and from 11,293 to 11,570 (Δ28) for a total of 2,583 bp removed from the T7 genome. Therefore, almost 2.6 Kb of foreign DNA can be inserted into these vectors, while still being no larger than the wild type T7 genome. Similar techniques or linkers are utilized to introduce other sites into either the Bam HI site or other convenient sites within T7 or other lethal phage.

### Further Increasing the Capacity of T7-Derived Vectors.

In addition to the above there are other areas of the T7 genome that have been found deleted in certain natural mutants such as T7-LG4 described below. These deletions are combined with T101 or T102 to make vectors that have an even larger capacity as follows.

In a first procedure, E. coli BL 21 cells are coinfected with T7-T101::GC2B and T7-LG4, a mutant of T7 lacking the segment from 5845 to 7879 bp on the T7 map for a total deletion of 2,034 bp, resulting in production of a vector having all three deletions. Thus it has 4,617 bp missing from the wild type T7 genome. Surprisingly, it is then possible to successfully package the resulting vector, which is hereafter referred to as T7-T104. As noted, the Δ1306 and T7-LG4 deletions together result in a loss of 4.3 Kb from the wild type T7 genome. However, the presence of the GC2B insert (approximately 1.7 Kb) results in T7-T104 being only about 2.6 Kb smaller than wild type T7.

Next, an evaluation is made as to whether the presence of the GC2B insert is necessary for phage viability. Accordingly, T7-T104 DNA is digested with Eco R1, excising the GC2B insert while preserving the polylinker, in a process similar to that described above in the construction of T7-T102 from T7-T101::GC2B Subsequent ligation, packaging and restriction analysis identifies one vector characterized by theΔl306 and LG4 deletions and which also lacks the GC2B insert. This is now designated T7-T105. This demonstrates that the presence of the additional amount of DNA provided by an insert sequence is not required for viability of T7-T105.

In accordance with the present invention a broad spectrum of sequences can be inserted, replicated and recovered in homogeneous form using the vectors which have been prepared as described above. This example demonstrates the use of GC2B, while others which can and have also been used include species-specific sequences from Campylobacter jejuni.

### EXAMPLE 2

### COMPARATIVE HYBRIDIZATION ASSAYS

The experiments reported in this example compare the use of two recombinant vectors carrying a foreign DNA segment as DNA probes for species identification. Both vectors comprise the insert GC2B, described in Example 1. The first vector is pUC9. The second vector is T7-Δ1306, Δ28, also described in Example 1 and also carrying the GC2B insert.

The techniques used to isolate and purify the GC2B insert DNA from pUC9 are as described in Maniatis, et al., supra. and comprise isolation of plasmid DNA by lysozyme/phenol extractions, purification of plasmid DNA by CsCl gradient; buoyant density centrifugation; repurification by a second CsCl centrifugation; excision of the GC2B insert by digestion with Eco R1; separation and purification of the GC2B insert from the pUC9 vector by preparative agarose gel electrophoresis followed by extraction of the GC2B band from the agarose by electroelution. This electrophoresis purification step was then repeated to give GC2B insert DNA for labelling.

The GC2B insert is not purified from its T7-Δ1306, Δ28 vector, but rather is left in situ and both the insert and the vector DNA sequences are labelled together.

Both the purified GC2B insert DNA derived from the pUC9 plasmid vector, the T7-Δ1306,Δ28::GC2B are labelled with biotin. Details of preparation of filters with target sequences as well as the subsequent hybridization and washing steps are as described in Reference Example 1. Detection of biotin labelled hybridization was performed using a DETEK® signal generating system (Enzo Biochem, Inc., New York, New York) as described the accompanying literature. Target DNA was dotted with the same procedure as described in Reference Example 1 with the exception of the amounts of DNA dotted onto the filters. Table 2 reports the results of this experiment.

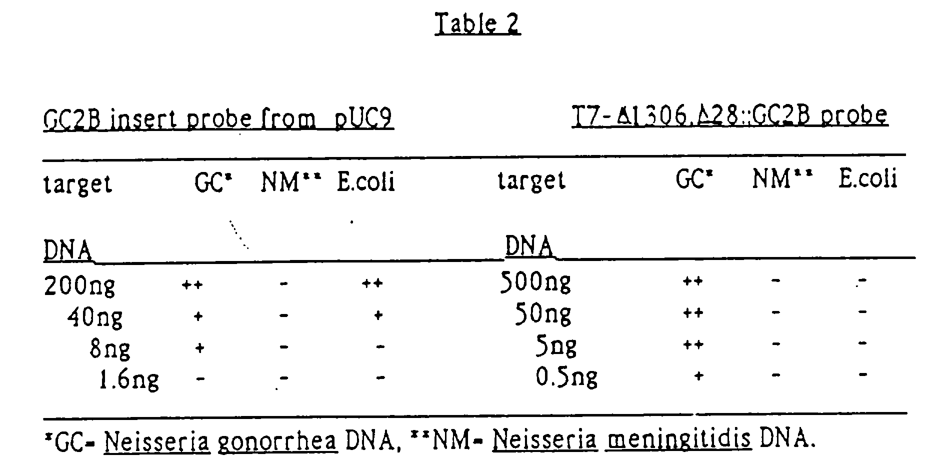

It can be seen from these data that there is extensive cross-reactivity with E. coli even when using the pUC9 plasmid derived GC2B insert DNA that had been extensively purified, whereas the T7-Δ1306, Δ28::GC2B vector shows no reactivity with E. coli DNA. At the same time the GC2B sequences in the T7-Δ1306,Δ28::GC2B vector are still completely functional in being able to recognize Neisseria gonorrhea DNA (from which the GC2B sequences were derived) apart from Neisseria meningitidis.

## Claims

1. A reagent composition for determining the presence or absence of a target nucleic acid in a sample, said composition comprising:
(a) a nucleic acid construct consisting essentially of:
(i) a nucleic acid vector which is a bacteriophage selected from the group consisting of T7-T102 which is a derivative of T7 having deletions spanning the regions from 836 to 3142 and from 11293 to 11570 base pairs of the T7 genome and having the restriction sites shown in Fig. 4; T7-T104 which is a derivative of T7 having deletions spanning the regions from 836 to 3142, from 5845 to 7879 and from 11293 to 11570 base pairs of the T7 genome and having the insert GC2B consisting of the DNA sequence inserts GC155 and GC1657 from N. gonorrhoeae as shown in Fig. 3; and T7-T105 which corresponds to T7-T104 wherein the insert GC2B has been deleted;
(ii) a segment of nucleic acid, which segment is sufficiently homologous to said target to hybridize substantially only with that target sequence and not with other sequences that may be present as contaminants in said sample; and
(iii) at least one labeling system component which is required for production of a detectable signal and is covalently bound to at least one of (i) or (ii);
and
(b) at least one additional component of said labeling system which is bindable with said labeling system component (iii) and renders said labeling system detectable (iii) when said labeling system component is not itself detectable.

2. The composition of claim 1 wherein said nucleic acid construct is prepared by a method comprising the steps of:
introducing into the vector of (i), a nucleic acid segment of (ii) to form said nucleic acid construct;
replicating the construct so-formed in a suitable host that is in a medium in which replication can occur;
eliminating the presence of any hybridizable nucleic acid sequence Which is not part of the nucleic acid sequence of the construct; and
recovering the nucleic acid construct consisting essentially of (i) and (ii).

3. The composition of claim 2 wherein the method comprises the additional step of intioducing, after lysis but before release of the bacteriophage nucleic acid from the bacteriophage coat such that the bacteriophage nucleic acid remains intact, a substance which degrades nucleic acids to an extent that they are no longer capable of hybridizing, but not protein, and said bacteriophage has a protein-containing coat which protects the bacteriophage nucleic acid such that it remains intact.

4. The composition of claim 2 wherein the vector is replicated In a bacterial cell and which method comprises the additional step of disrupting the membrane of said bacterial cell.

5. The composition of claim 2 wherein the method comprises the additional step of introducing, after lysis but before release of the bacteriophage nucleic acid from the bacteriophage coat such that the bacteriophage nucleic acid remains intact, a nuclease which degrades nucleic acids to an extent that they are no longer capable of hybridizing, but not protein, and said bacteriophage has a protein-containing coat which protects the bacteriophage nucleic acid such that it remains intact.

6. The composition of claim 5 wherein said protecting coat is a protein, lipoprotein or mucoprotein coat.

7. The composition of claim 5 wherein said nuclease is a deoxyribonuclease or ribonuclease.

8. The composition of claim 7 wherein said nuclease is a preparation comprising both a deoxyribonuclease and a ribonuclease.

9. A method of analyzing a sample for the presence or absence of a target nucleic acid therein, which method comprises:
(1) contacting said sample under conditions permissive of hybridization with a reagent composition comprising:
(a) a nucleic acid construct consisting essentially of:
(i) a nucleic acid vector as defined in claim 1
(ii) a segment of nucleic acid which segment is sufficiently homologous to said target to be detected to hybridize substantially only with that target sequence and not with other sequences that may be present as contaminants in said sample; and
(iii) at least one labeling system component required for production of a detectable signal covalently bound to at least one of (i) or (ii); and
(b) at least one additional component of said labeling system which is bindable with said labeling system component (iii) and renders said labeling system component (iii) detectable where said component is not itself detectable; and
(2) observing the presence or absence of any detectable response.

10. The method of claim 9 wherein the nucleic acid construct of the reagent composition is prepared by a method comprising the steps of:
introducing into the vector of (i) a nucleic acid segment of (ii) to form said nucleic acid construct;
replicating the construct so-formed in a suitable host that is in a medium in which replication can occur;
eliminating the presence of any hybridizable nucleic acid sequence which is not part of the nucleic acid sequence of the construct; and
recovering the nucleic acid construct consisting essentially of (i) and (ii).

11. The method of claim 10 wherein said construct is replicated in a bacterial cell and which method comprises the additional step of disrupting the membrane of said bacterial cell after lysis but before release of the phage DNA from the phage coat such that the phage DNA remains intact.

12. The method of claim 10 which comprises the additional step of introducing, after lysis but before release of the bacteriophage nucleic acid from the bacteriophage coat such that the bacteriopnage nucleic acid remains intact, a nuclease which degrades nucleic acid to an extent that they are no longer capable of hybridizing, but not protein, and said nucleic acid vector is a virulent bacteriophage having a protein-containing coat which protects the bacteriophage nucleic acid thereof such that it remains intact.

13. The method of claim 12 wherein said protecting coat is a protein, lipoprotein or mucoprotein coat.

14. The method of claim 12 wherein said nuclease is a deoxyribonuclease or ribonuclease.

15. The method of claim 12 wherein said nuclease is a preparation comprising both a deoxyribonuclease and a ribonuclease.

## Patentansprüche

1. Reagenzzusammensetzung zur Bestimmung der Gegenwart oder Abwesenheit einer Zielnucleinsäure in einer Probe, wobei die Zusammensetzung umfaßt:
(a) ein Nucleinsäure-Konstrukt, im wesentlichen bestehend aus:
(i) einem Nucleinsäurevektor, der ein Bakteriophage ist, ausgewählt aus T7-T102, der ein Derivat von T7 ist, mit Deletionen, die die Bereiche von 836 bis 3142 und von 11293 bis 11570 Basenpaaren des T7-Genoms umfassen, und mit den in Fig. 4 gezeigten Restriktionsstellen; T7-T104, der ein Derivat von T7 ist, mit Deletionen, die die Bereiche von 836 bis 3142, von 5845 bis 7879 und von 11293 bis 11570 Basenpaaren des T7-Genoms umfassen, und mit der Insertion GC2B bestehend aus den DNA-Sequenz-Insertionen GC155 und GC1657 von N. gonorrhoeae, wie in Fig. 3 gezeigt; und T7-T105, der T7-T104 entspricht, in dem die Insertion GC2B deletiert worden ist;
(ii) einem Nucleinsäuresegment, wobei das Segment ausreichend homolog zu der Zielnucleinsäure ist, so daß es im wesentlichen nur mit jener Zielsequenz hybridisiert und nicht mit anderen Sequenzen, die als Verunreinigungen in der Probe vorhanden sein können; und
(iii) zumindest eine Markierungssystemkomponente, die für die Produktion eines nachweisbaren Signals erforderlich ist und kovalent an zumindest eine Nucleinsäure von (i) oder (ii) gebunden ist;
und
(b) zumindest eine zusätzliche Komponente des Markierungssystems, die an die Markierungssystemkomponente (iii) binden kann und das Markierungssystem (iii) nachweisbar macht, wenn die Markierungssystemkomponente selbst nicht nachweisbar ist.

2. Zusammensetzung nach Anspruch 1, wobei das Nucleinsäurekonstrukt nach einem Verfahren hergestellt wird, umfassend die Schritte:
Einbringen eines Nucleinsäuresegments von (ii) in den Vektor von (i) zur Erzeugung des Nucleinsäurekonstrukts, Replikation des so erzeugten Konstrukts in einem geeigneten Wirt in einem Medium, in welchem Replikation stattfinden kann;
Entfernen jeder beliebigen hybridisierbaren Nucleinsäuresequenz, die nicht Teil der Nucleinsäuresequenz des Konstrukts ist; und Gewinnen des Nucleinsäurekonstrukts, das im wesentlichen aus (i) und (ii) besteht.

3. Zusammensetzung nach Anspruch 2, wobei das Verfahren den zusätzlichen Schritt umfaßt, eine Substanz nach der Lyse, aber vor der Freisetzung der Bakteriophagen-Nucleinsäure aus der Bakteriophagenhülle, so daß die Bakteriophagen-Nucleinsäure intakt bleibt, einzubringen, die Nucleinsäuren, aber nicht Protein, in einem Maß abbaut, daß sie nicht länger in der Lage sind zu hybridisieren, wobei der Bakteriophage eine Protein-enthaltende Hülle hat, die die Bakteriophagen-Nucleinsäure schützt, so daß sie intakt bleibt.

4. Zusammensetzung nach Anspruch 2, wobei der Vektor in einer Bakterienzelle repliziert wird, und wobei das Verfahren den zusätzlichen Schritt des Aufbrechens der Membran der Bakterienzelle umfaßt.

5. Zusammensetzung nach Anspruch 2, wobei das Verfahren den zusätzlichen Schritt umfaßt, eine Nuclease nach der Lyse, aber vor der Freisetzung der Bakteriophagen-Nucleinsäure aus der Bakteriophagenhülle, so daß die Bakteriophagen-Nucleinsäure intakt bleibt, einzubringen, die Nucleinsäuren, aber nicht Protein, in einem Maß abbaut, daß sie nicht länger in der Lage sind zu hybridisieren, wobei der Bakteriophage eine Protein-enthaltende Hülle hat, die die Bakteriophagen-Nucleinsäure schützt, so daß sie intakt bleibt.

6. Zusammensetzung nach Anspruch 5, wobei die schützende Hülle eine Protein-, Lipoprotein- oder Mucoproteinhülle ist.

7. Zusammensetzung nach Anspruch 5, wobei die Nuclease eine Desoxyribonuclease oder Ribonuclease ist.

8. Zusammensetzung nach Anspruch 7, wobei die Nuclease ein Präparat ist, das sowohl eine Desoxyribonuclease als auch eine Ribonuclease umfaßt.

9. Verfahren zur Analyse einer Probe auf die Gegenwart oder Abwesenheit einer Zielnucleinsäure darin, wobei das Verfahren umfaßt:
(1) das Inkontaktbringen der Probe unter Bedingungen, die eine Hybridisierung erlauben, mit einer Reagenzzusammensetzung, umfassend:
(a) ein Nucleinsäure-Konstrukt, im wesentlichen bestehend aus:
(i) einem Nucleinsäurevektor, wie in Anspruch 1 definiert,
(ii) einem Nucleinsäuresegment, wobei das Segment ausreichend homolog zu der nachzuweisenden Zielnucleinsäure ist, so daß es im wesentlichen nur mit jener Zielsequenz und nicht mit anderen Sequenzen hybridisiert, die als Verunreinigungen in der Probe vorhanden sein können; und
(iii) zumindestens eine Markierungssystemkomponente, die für die Erzeugung eines nachweisbaren Signals erforderlich ist, und die kovalent an zumindest eine Nucleinsäure von (i) oder (ii) gebundenen ist; und
(b) mindestens eine zusätzliche Komponente des Markierungssystems, die an die Markierungssystemkomponente (iii) binden kann und die Markierungssystemkomponente (iii) nachweisbar macht, wenn die Komponente selbst nicht nachweisbar ist; und
(2) Feststellung des Vorhandenseins oder Nichtvorhandenseins einer beliebigen nachweisbaren Antwort.

10. Verfahren nach Anspruch 9, wobei das Nucleinsäurekonstrukt der Reagenzzusammensetzung nach einem Verfahren hergestellt wird, das die folgenden Schritte umfaßt:
Einbringen eines Nucleinsäuresegments von (ii) in den Vektor von (i), zur Erzeugung des Nucleinsäurekonstrukts;
Replikation des so erzeugten Konstrukts in einem geeigneten Wirt in einem Medium, in welchem die Replikation stattfinden kann;
Entfernen jeder hybridisierbaren Nucleinsäuresequenz, die nicht Teil der Nucleinsäuresequenz des Konstrukts ist; und
Gewinnen des Nucleinsäurekonstrukts, das im wesentlichen aus (i) und (ii) besteht.

11. Verfahren nach Anspruch 10, wobei das Konstrukt in einer Bakterienzelle repliziert wird, und wobei das Verfahren den zusätzlichen Schritt des Aufbrechens der Membran der Bakterienzelle nach der Lyse, aber vor der Freisetzung der Phagen-DNA aus der Phagenhülle umfaßt, so daß die Phagen-DNA intakt bleibt.

12. Verfahren nach Anspruch 10, welches den zusätzlichen Schritt umfaßt, eine Nuclease nach der Lyse, aber vor der Freisetzung der Bakteriophagen-Nucleinsäure aus der Bakteriophagenhülle, so daß die Bakteriophagen-Nucleinsäure intakt bleibt, einzubringen, die Nucleinsäuren, aber nicht Protein, in einem Maß abbaut, daß sie nicht länger in der Lage sind zu hybridisieren, wobei der Nucleinsäurevektor ein virulenter Bakteriophage ist, der eine Protein-enthaltende Hülle hat, die die Bakteriophagen-Nucleinsäure davor schützt, so daß sie intakt bleibt.

13. Verfahren nach Anspruch 12, wobei die schützende Hülle eine Protein-, Lipoprotein- oder Mucoproteinhülle ist.

14. Verfahren nach Anspruch 12, wobei die Nuclease eine Desoxyribonuclease oder eine Ribonuclease ist.

15. Verfahren nach Anspruch 12, wobei die Nuclease ein Präparat ist, das sowohl eine Desoxyribonuclease als auch eine Ribonuclease umfaßt.

## Revendications

1. Une composition de réactif pour déterminer la présence ou l'absence d'un acide nucléique cible dans un échantillon, ladite composition comportant:
(a) un produit de recombinaison d'un acide nucléique comportant :
(i) un vecteur d'acide nucléique qui est un bactériophage choisi dans le groupe constitué du T7-T102, qui est un dérivé de T7 présentant des suppressions recouvrant les régions des paires de base de 836 à 3142 et de 11293 à 11570 du génome T7 et qui présente les sites de restriction représentés sur la figure 4, du T7-T104 qui est un dérivé de T7 présentant des suppressions recouvrant des régions des paires de base de 836 à 3142, de 5845 à 7879 et de 11293 à 11570 du génome T7 et possédant l'insert GC2B constitué des inserts GC155 et GC1657 de la séquence ADN provenant du N-gonorrhoeae, comme représenté sur la figure 3, et du T7-T105 qui correspond à T7-T104 dans lequel l'insert GC2B a été supprimé;
(ii) un segment d'acide nucléique, ce segment étant suffisamment homologue à ladite cible pour ne s'hybridiser essentiellement qu'avec cette séquence cible et pas avec d'autres séquences qui peuvent être présentes en tant qu'agents contaminants dans ledit échantillon;
(iii) au moins un composant de système de marque qui est nécessaire pour la production d'un signal susceptible d'être détecté et est lié, de façon covalente, à au moins à un des (i) ou (ii);
et
(b) au moins un composant supplémentaire dudit système de marquage susceptible de se lier avec ledit composant de système de marquage (iii) et rendre ainsi ledit système de marquage (iii) susceptible d'être détecté, lorsque ledit composant de marquage n'est pas lui-même susceptible d'être détecté.

2. La composition de la revendication 1, dans laquelle ledit produit de recombinaison de l'acide nucléique est préparé par un procédé comportant les étapes:
d'introduire dans le vecteur de (i) un segment d'acide nucléique (ii) pour former ledit produit de recombinaison nucléique;
de répliquer le produit de recombinaison ainsi formé dans un hôte approprié qui se trouve dans un milieu dans lequel une réplication peut se produire;
d'éliminer la présence d'une séquence d'acide nucléique, susceptible d'être hybridée, qui ne fait pas partie de la séquence d'acide nucléique du produit de recombinaison; et
de récupérer le produit de recombinaison de l'acide nucléique, qui est constitué essentiellement de (i) et de (ii).

3. La composition de la revendication 2, dans laquelle le procédé comprend l'étape supplémentaire d'introduire, après la lyse mais avant la libération de l'acide nucléique du bactériophage provenant de l'enveloppe du bactériophage, de façon que l'acide nucléique du bactériophage reste intact, une substance qui dégrade les acides nucléiques dans une mesure telle qu'ils ne sont plus aptes à l'hybridation, pas une protéine, et que ledit bactériophage possède une enveloppe renfermant une protéine, qui protège l'acide nucléique du bactériophage de sorte qu'il reste intact.

4. La composition de la revendication 2, dans laquelle le vecteur est répliqué dans une cellule bactérienne tandis que ce procédé comporte l'étape supplémentaire de rompre la membrane de ladite cellule bactérienne.

5. La composition de la revendication 2, dans laquelle le procédé comporte l'étape supplémentaire d'introduire, après la lyse mais avant la libération de l'acide nucléique du bactériophage provenant de l'enveloppe du bactériophage de sorte que l'acide nucléique du bactériophage reste intact, une nucléase qui dégrade les acides nucléiques dans une mesure telle qu'ils ne sont plus capables d'hybridation, mais pas une protéine, et que ledit bactériophage possède une enveloppe renfermant une protéine, qui protège l'acide nucléique du bactériophage de façon qu'il reste intact.

6. La composition de la revendication 5, dans laquelle ladite enveloppe de protection est une enveloppe à base de protéine, de lipoprotéine ou de mucoprotéine.

7. La composition de la revendication 5, dans laquelle ladite nucléase est une désoxyribonucléase ou une ribonucléase.

8. La composition de la revendication 7, dans laquelle ladite nucléase est une préparation comportant à la fois une désoxyribonucléase et une ribonucléase.

9. Procédé pour analyser un échantillon, pour y déterminer la présence ou l'absence d'un acide nucléique cible, ce procédé comportant:
(1) la mise en contact dudit échantillon sous des conditions susceptibles de permettre l'hybridation avec une composition de réactif comportant:
(a) un produit de recombinaison de l'acide nucléique constitué essentiellement de:
(i) un vecteur de l'acide nucléique tel que défini dans la revendication 1;
(ii) un segment de l'acide nucléique, ce segment étant suffisamment homologue par rapport à ladite cible à détecter, afin de ne s'hybridiser essentiellement qu'avec la séquence cible et pas avec d'autres séquences qui peuvent être présentes sous la forme d'agents contaminants dans ledit échantillon; et
(iii) au moins un composant d'un système de marquage, nécessaire pour la production d'un signal susceptible d'être détecté, lié de façon covalente à un des (i) ou (ii); et
(b) au moins un composant supplémentaire dudit système de marquage, et susceptible de se lier avec ledit composant du système de marquage (iii) de rendre ledit composant du système de marquage (iii) susceptible d'être détecté là où ledit composant n'est pas lui-même susceptible d'être détecté; et
(2) l'observation de la présence ou de l'absence de réponse quelconque, susceptible d'être détectée.

10. Procédé de la revendication 9, dans lequel ledit produit de recombinaison de l'acid nucléique de la composition de reactif est préparé par un procédé comportant les étapes
d'introduire dans le vecteur de (i) un segment d'acide nucléique (ii) pour former ledit produit de recombinaison nucléique;
de répliquer le produit de recombinaison ainsi formé dans un hôte approprié qui se trouve dans un milieu dans lequel une réplication peut se produire;
d'éliminer la présence d'une séquence d'acide nucléique, susceptible d'être hybridée, qui ne fait pas partie de la séquence d'acide nucléique du produit de recombinaison; et
de récupérer le produit de recombinaison de l'acide nucléique, qui est constitué essentiellement de (i) et de (ii).

11. Le procédé de la revendication 10, dans laquelle ledit produit de recombinaison est répliqué dans une cellule bactérienne, ce procédé comportant l'étape supplémentaire de briser la membrane de ladite cellule bactérienne après la lyse mais avant la libération de l'ADN du phage à partir de l'enveloppe du phage, de sorte que 1'ADN du phage reste intacte.

12. Le procédé de la revendication 10, qui comporte l'étape supplémentaire d'introduire, après la lyse mais avant la libération de l'acide nucléique du bactériophage provenant de l'enveloppe du bactériophage de sorte que l'acide nucléique du bactériophage reste intact, une nucléase qui dégrade les acides nucléiques dans une mesure telle qu'ils ne sont plus capables d'hybridation, mais pas un protéine, et que ledit vecteur dàcide nucléique est un bactériophage virulent possédant une envelope renfermant une protéine, qui protège l'acide nucléique du bactériophage de façon qu'il reste intact.

13. Le procédé de la revendication 12, dans lequel ladite enveloppe protectrice est une enveloppe à base de protéine, de lipoprotéine, ou de mucoprotéine.

14. Le procédé de la revendication 12, dans laquelle ladite nucléase n'est une désoxyribonucléase ou une ribonucléase.

15. Le procédé de la revendication 14, dans lequel ladite nucléase est une préparation comportant à la fois une désoxyribonucléase et une ribonucléase.
